# EUROPEAN PATENT APPLICATION

(11) **EP 3 369 383 A1**
(43) Date of publication of application: **05.09.2018**
(21) Application number: 17158823.9
(22) Date of filing: 02.03.2017
(51) Int. Cl.: A61B 8/08, A61B 8/12, A61B 8/00, B06B 1/02, G01N 29/24

(54) **ULTRASOUND DEVICE**

(71) Applicant: Koninklijke Philips N.V., 5656 AE Eindhoven (NL)
(72) Inventor: JACOBS, Egbertus Reinier, 5656 AE Eindhoven (NL)
(74) Representative: de Haan, Poul Erik

(57) **Abstract**

An ultrasound device comprises an ultrasound head at a distal end of the shaft. Electrical circuitry for driving the ultrasound head includes circuit components mounted on a flexible carrier so that they do not require a fully rigid part of the device. The transducer aperture can then be made as large as possible for a given size of rigid substrate. A reduced rigid substrate length improves maneuverability of the device.

## Description

### FIELD OF THE INVENTION

This invention relates to devices which incorporate ultrasound imaging at the tip of a shaft.

### BACKGROUND OF THE INVENTION

Inter-cardiac ultrasound (ICE) catheters are well known. They typically incorporate piezoelectric or single crystal ultrasound elements at the catheter tip, for performing localized imaging.

The catheter shaft needs to be flexible, to enable the catheter to be steered within the arteries and within the heart. However, the ultrasound head needs to be formed as a rigid structure, and it is typically formed at a rigid tip part of the catheter.

In designs using a piezoelectric transducer or a single crystal ultrasound element, the catheter has a large aperture compared to the rigid tip length (i.e. the rigid tip is used only for the ultrasound head) and there are no active devices in the tip. This enables maximal maneuverability in the heart while having a good image performance and penetration depth.

However, there is a desire to make use of capacitive micro-machined ultrasound transducers (CMUTs). CMUT devices are becoming increasingly popular because they can offer excellent bandwidth and acoustic impedance characteristics, which makes them the preferable over e.g. piezoelectric transducers. Vibration of the CMUT membrane can be triggered by applying pressure (for example using ultrasound) or can be induced electrically.

CMUT devices enable an array of transducers to be formed, for example so that electronic beam-forming in 2D or 3D becomes possible. The transducers are then produced in arrays with transducer element sizes of λ/2 where λ is the wavelength of the used ultrasound frequency in body tissue.

CMUT array devices require local integrated circuitry (in particular ASICs) to enable operation of the CMUT elements. For example, high voltage pulses of a pulser circuit and a high voltage DC bias are generated by a probe circuit, which is typically an application specific integrated circuit (ASIC) which is located within the ultrasound probe, i.e. at the probe location. The ASIC facilitates both transmission and reception modes of the device. In reception mode, changes in the membrane position cause changes in electrical capacitance, which can be registered electronically. In transmission mode, applying an electrical signal causes vibration of the membrane.

Besides the ASICs, several capacitors are also needed to enable a stable power supply to the ASIC and CMUT. These capacitors are typically rather large.

Most interconnect techniques for assembly of the ASICs and capacitors are on rigid substrates. By providing these locally with the transducer element, these rigid substrates consume a lot of the ultrasound aperture, thus resulting in a reduced image performance and penetration depth and/or they extend the rigid tip length, thus reducing maneuverability.

Another problem is the cabling leading to the ultrasound head. A large number of micro coaxial cables are used, and these cables must all be terminated at the same rigid substrate. They also consume some of the aperture space or extend the rigid tip length.

There is therefore a need to maximize the transducer head aperture while minimizing the rigid tip length in designs which require local ultrasound probe circuitry such as ASICs or passive circuit elements.

### SUMMARY OF THE INVENTION

The invention is defined by the claims.

According to examples in accordance with an aspect of the invention, there is provided an ultrasound device comprising:
a shaft;
an ultrasound head at a distal end of the shaft;
electrical circuitry for driving the ultrasound head comprising circuit components; and
a flexible component carrier extending from the ultrasound head,
wherein the circuit components are carried on the flexible component carrier.

This device make use of a flexible carrier on which electrical components may be carried, so that they do not require a fully rigid part of the device. These components are thus in a bendable area of the device. There may be circuit component on a rigid substrate at the ultrasound head as well, but there are at least some components on the flexible component carrier, which would otherwise need to be located at the ultrasound head. The circuit components, such as capacitors and/or ASICs, as well as the cable terminations are thus provided in the flexible area of the device, on the flexible carrier.

By moving components off a rigid part of the device where the ultrasound head is located, the transducer aperture is as large as possible, giving improved imaging performance, such as penetration depth, resolution, etc. The flexible carrier can bend with the shaft and does not contribute to the rigid tip length. A reduced rigid tip length improves maneuverability of the device, for example in the atria of the heart in the case of an imaging catheter, where only limited space is present.

The ultrasound head for example comprises a CMUT transducer array.

In one example, the circuit components comprise one or more ASICs. These may be form part of the transmit and receive circuitry of the transducer head.

The circuit components may additionally or alternatively comprise one or more resistors and/or capacitors.

The flexible component carrier may comprise a flexible circuit board. In this way, the components may be mounted on the circuit board in conventional manner, and the populated board can then be connected to the transducer head (and to connection wires leading along the shaft).

In one example, the flexible component carrier comprises an array of separate carrier components arranged side by side. They run along the shaft direction. This avoids the need for a wide carrier, which would be resistant to bending across the width direction. By having multiple carriers with a small aspect ratio (e.g. less than 2) they can individually bend in all directions and move relative to each other to avoid kinking.

In another example, the flexible component carrier comprises a helically wound carrier track. This provides another way to avoid kinking.

In another example, the flexible component carrier comprises a helically twisted carrier track. This provides another way to avoid kinking which requires a reduced additional length of the flexible carrier compared to a helically wound solution.

The device may further comprise a set of connection wires which extend along the shaft and connect to the flexible component carrier at a first, proximal, end opposite to the ultrasound head.

The flexible component carrier thus functions as an interface between the ultrasound head and the connection cables, which for example comprise a set of coaxial cables.

The first end of the flexible component carrier may comprise a set of longitudinally relatively displaced fingers to each of which a subset of the set of connection wires connect. In this way, it is avoided that all connections to the flexible carrier are at the same longitudinal position, so that the space occupied can be spread along the length of the shaft. The fingers may also be coiled around to occupy less space.

The flexible component carrier may comprise a polyimide or liquid crystal polymer flex.

The device for example comprises a catheter.

### BRIEF DESCRIPTION OF THE DRAWINGS

Examples of the invention will now be described in detail with reference to the accompanying drawings, in which:
Figure 1 shows a conventional configuration for an ultrasound transducer catheter tip and also shows a configuration in accordance with the general teaching of the invention;
Figure 2 shows a flexible carrier and shows different bending directions;
Figure 3 shows a first approach to avoid kinking of the flexible carrier;
Figure 4 shows a cross section through the catheter of Figure 3 at an arbitrary point along the length of the flexible carrier;
Figure 5 shows a second approach to avoid kinking of the flexible carrier;
Figure 6 shows a third approach to avoid kinking of the flexible carrier; and
Figure 7 shows a cable termination approach.

### DETAILED DESCRIPTION OF THE EMBODIMENTS

The invention provides an ultrasound device which comprises an ultrasound head at a distal end of the shaft. Electrical circuitry for driving the ultrasound head includes circuit components mounted on a flexible carrier so that they do not require a fully rigid part of the device. The transducer aperture can then be made as large as possible for a given size of rigid substrate. A reduced rigid substrate length improves maneuverability of the device.

The invention will be described with reference to ultrasound imaging at the tip of a catheter.

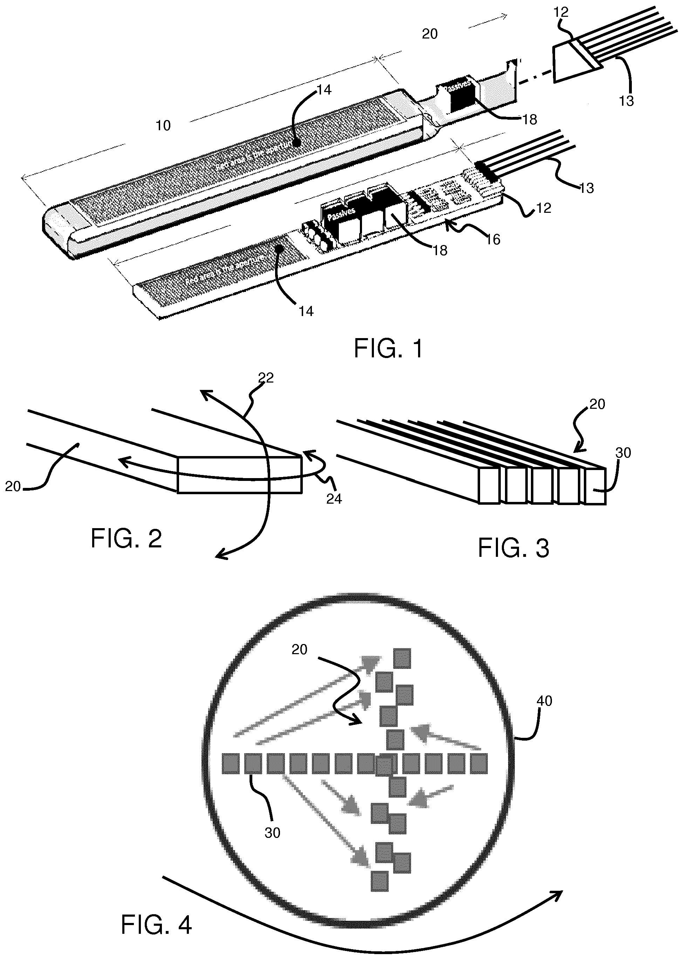

Figure 1 shows a conventional configuration for an ultrasound transducer catheter tip and a configuration in accordance with the general teaching of the invention.

In the conventional configuration, there is a rigid substrate 10 which comprises a bank 12 of electrical connectors at one end for connection to an array of coaxial cables 13. At the other end is the transducer head 14. The size of the transducer head defines the aperture of the transducer. In the example shown, the transducer head occupies about 50% of the area of the rigid substrate. The rigid substrate also carries integrated circuits 16 and passive components 18 which together form the driving circuitry (transmit and receive) for the transducer head 14.

The approach of the invention makes use of almost the full rigid substrate 10 for the transducer head 14. The components, such as the passive component 18, is provided on a flexible carrier 20, such as a flexible printed circuit board. At the end of the flexible carrier, the connections 12 to the coaxial cables are made.

The flexible carrier provides electrical connections between the coaxial cables and the components, and connections between the components and the transducer head, and any direct connection (such as ground) that are needed between the coaxial cables and the transducer head. The transducer head is mounted on the flexible carrier. However, the mounting of the transducer head at the end of the flexible carrier renders the end of the flexible carrier rigid, whereas the smaller components mounted proximally of the transducer head still enable some flexibility to be retained.

The structure of Figure 1 is provided along a shaft, which in this example is a catheter shaft, with the wires, flexible carrier and transducer head within the catheter wall.

The gain in transducer aperture for the same size rigid substrate 10 can clearly be seen. As an alternative, a smaller rigid substrate, and hence more easily maneuvered device, may be provided for a given required transducer aperture.

Almost the complete rigid substrate area can be used for placing active CMUT devices and thus extending the aperture.

There are two main options for the CMUT arrangement.

A first option is to provide ASIC functionality below the CMUT drums, resulting in a monolithically integrated approach. The CMUT is then processed using thin film technology on top of an ASIC wafer. In this case, part or all of the ASIC functionality is provided on the rigid tip part with the CMUT drums. Additional functionality, such as passive components or further integrated circuits are then provided on the flexible carrier.

A second option is to have separate ASIC functionality and CMUT drums. In this case, the ASIC functionality may be provided on the flexible carrier together with the passive components.

In all examples, the transducer provided on the rigid substrate basically comprises an acoustic generator and receiver (which may be a CMUT cell, or a PZT device or a single crystal device). Depending on the implementation, there may be no ASIC functionality, part of the ASIC functionality, or the full ASIC functionality for a monolithic solution, on the rigid substrate. The flexible carrier will typically incorporate the remaining ASIC functionality and the passive components, such as capacitors, resistors and inductors.

The technology for mounting active and passive components on a flexible substrate is well known and is for example widely used in the field of wearable electronics and other flexible applications. Flexible substrates are known for use as interconnect boards and other electronic components.

Typically, components are first soldered onto the flexible substrate by soldering (reflow soldering, wave soldering, etc.).

The cable termination process is the second process which takes place, for connecting the coaxial cables to the flexible board.

Finally, the flexible board is attached to the rigid transducer chip by a bonding process, such as wire bonding, thermo-compression bonding, ACF bonding or soldering.

After this step the total tip assembly is ready and can be put into a catheter shaft. For this, the cables are guided through the catheter shaft until the complete cable including the flexible substrate with the passive components is in the catheter shaft.

The purpose of the flexible carrier is to enable circuit components to be provided at a flexible part of the catheter. However, a flexible substrate is typically only flexible in certain directions.

Figure 2 shows a flexible carrier 20. A first bending direction 22 can easily be formed (about an axis in the plane of the carrier across its width). However, a second bending direction 24 (about an axis perpendicular to the plane of the carrier) results in kinking.

There are various ways to provide a flexible carrier design which avoids this kinking issue.

Figure 3 shows a first approach by which the flexible carrier is formed as an array of separate carrier components 30 arranged side by side. Each carrier component 30 runs along the length of the shaft can carried circuit tracks and/or circuit components (not shown). The different components are fixed in position relative to each other where they connect to the rigid substrate. However, beyond the rigid substrate, they are free to move relative to each other, so that bending across the width (arrow 24 in Figure 2) becomes possible without kinking.

Each carrier component for example has an aspect ratio of its cross sectional shape (in cross section perpendicular to the length direction) of less than 3, for example less than 2.

Figure 4 shows a cross section through the catheter at an arbitrary point along the length of the flexible carrier. It shows the catheter wall 40 and a set of the carrier components 30 which have become displaced from their aligned positions.

Bending around the direction 24 shown in Figure 2 will result in a re-arranged track sequence because the individual components can move individually. Figure 4 shows that bending in the direction 24 will cause re-orientation of the set of components towards a 90 degree rotation. Before bending, the components are aligned in a horizontal direction, but when the catheter is bent, they have a tendency to move to the neutral line of the bending radius, which is vertical for the bending direction 24.

The components may be formed on individual ones of the carrier components. Alternatively, larger components, or components which need to connect to conducting lines on multiple carrier components, may connect to multiple carrier components. In this case, relative movement of those carrier components is not possible at the location of the large component, but the component is stiff in any case and very short. Therefore, the kinking risk is not present for these sections.

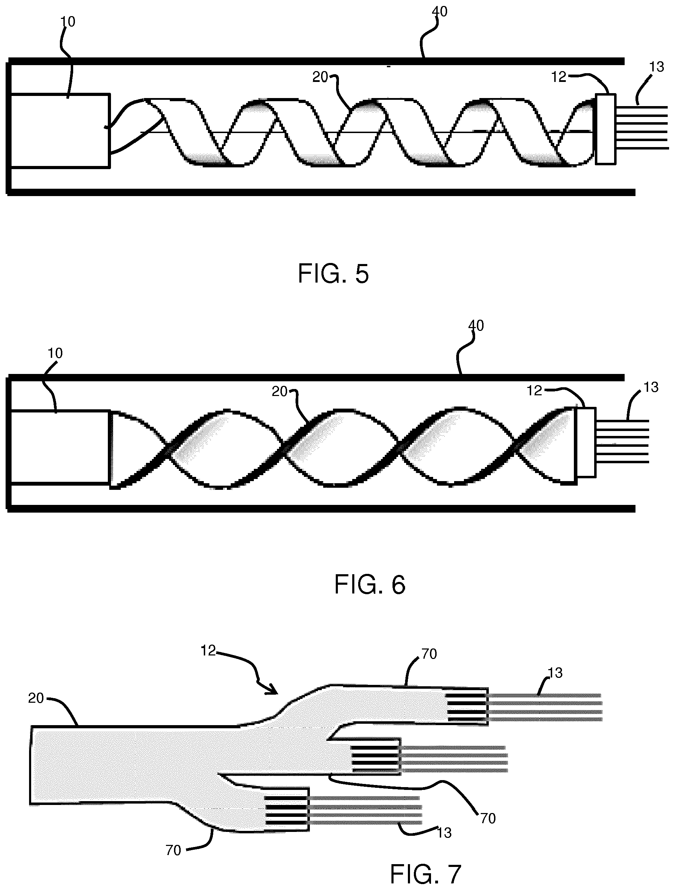

Figure 5 shows a second approach by which the flexible carrier 20 is wound into a helical spiral carrier track. This will of course require additional length of the flexible carrier, but relaxes the strain on the flexible carrier tremendously. The coiled/spiraled flexible carrier behaves like a close wound spring and is very easy to bend in all direction.

For the larger and rigid components on the flexible carrier, flat sections may be provided along the length axis of the carrier to accommodate them, since at these locations the flexible carrier cannot follow a curved path. Again, the components cannot bend, but due to the short length and the relatively large bending radius, this does not cause a problem. The bending force needed for deformation of the flexible carrier is particularly low in this example, since there is always a nearby carrier location where the carrier is at the optimal orientation for local bending (i.e. with bending in the direction of arrow 22 of Figure 2).

Figure 6 shows a third approach by which the flexible carrier 20 is wound into a helically twisted carrier track. This requires a reduced additional length of the flexible carrier compared to the design of Figure 5. The flexible carrier is twisted by a few strokes over the axial direction of the catheter, allowing for some degree of bendability without kinking.

By way of example only, the rigid substrate of the transducer head for example has a width of 2 to 4 mm and a length of 10 to 50 mm. For a catheter application, the catheter is typically 2.3 to 4 mm in diameter. However, for other applications a larger shaft may be present.

For a catheter application, there is typically a bendable end section of around 50 mm which is where the flexible carrier is mounted. The flexible carrier will have a length which depends on the desired components to be mounted, and it may for example be in the range of 1 to 5 mm wide and 12 to 100 micron thick. The length will also depend on the solution chosen for avoiding kinking. The flexible carrier is for example is in the bendable end section of the catheter but the connections to the wires may be in the non-bendable section.

As explained above, another issue with known designs is that the cable terminations occupy a significant space and they are at one longitudinal position along the catheter.

There is a large number of cables needed to make a CMUT array catheter so that a lot of space is normally consumed for termination of the cables. The connection wires are generally formed as coaxial cables, and they require two terminations per cable, making this a very complex step.

There may for example be between 3 and 50 wires to which connections are made. There may be multiplexing in the probe to reduce the number of wires or else there may be wires for each transducer element. Thus, the number of wires will depend on the multiplexing solution adopted. Such multiplexing will be implemented by the ASIC of the probe, which itself may be mounted on the flexible carrier or be a monolithic part of the transducer head as explained above.

Typically, the ground is common for all coaxial cables and is soldered by hot bar soldering, where one large piece of solder connects all cables. This requires a lot of space and is hard to accommodate in the catheter shaft, where only a limited diameter is available.

Figure 7 shows an approach by which the cables are divided into groups, and they are connected to the flexible carrier in a staggered way.

The connection wires which extend along the shaft and connect to the flexible component carrier 20 at a first proximal end (opposite to the ultrasound head). This first proximal end of the flexible component carrier is shown in Figure 7, and it shows a set of longitudinally relatively displaced fingers 70 to each of which a subset of the set of connection wires 13 connect.

The multiple finger shape can be folded into the diameter of the catheter shaft, and the different connection areas are then at different longitudinal positions along the catheter. The coaxial connection region can then even be located in a flexible part of the catheter.

The invention can be applied to all catheter-like ultrasound imaging products, which make use of passive or active circuit components and need to be as flexible as possible.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An ultrasound device comprising:
a shaft;
an ultrasound head (14) at a distal end (10) of the shaft;
electrical circuitry (16, 18) for driving the ultrasound head comprising circuit components; and
a flexible component carrier (20) extending from the ultrasound head,
wherein the circuit components are carried on the flexible component carrier (20).

2. A device as claimed in claim 1, wherein the ultrasound head (14) comprises a CMUT transducer array.

3. A device as claimed in any preceding claim, wherein the circuit components comprise one or more ASICs (16).

4. A device as claimed in any preceding claim, wherein the circuit components comprise one or more resistors and/or capacitors (18).

5. A device as claimed in any preceding claim, wherein the flexible component carrier (20) comprises a flexible circuit board.

6. A device as claimed in any preceding claim, wherein the flexible component carrier (20) comprises an array of separate carrier components (30) arranged side by side.

7. A device as claimed in any one of claims 1 to 5, wherein the flexible component carrier (20) comprises a helically wound carrier track.

8. A device as claimed in any one of claims 1 to 5, wherein the flexible component carrier (20) comprises a helically twisted carrier track.

9. A device as claimed in any preceding claim, further comprising a set of connection wires (13) which extend along the shaft and connect to the flexible component carrier (20) at a first, proximal, end opposite to the ultrasound head.

10. A device as claimed in claim 9, wherein the first, proximal, end of the flexible component carrier comprises a set of longitudinally relatively displaced fingers (70) to each of which a subset of the set of connection wires (13) connect.

11. A device as claimed in any preceding claim, wherein the flexible component carrier (20) comprises a polyimide or liquid crystal polymer flex.

12. A device as claimed in any preceding claim, comprising a catheter (40).
